# EUROPEAN PATENT APPLICATION

(11) **EP 4 574 176 A2**
(43) Date of publication of application: **25.06.2025**
(21) Application number: 25173139.4
(22) Date of filing: 05.06.2019
(51) Int. Cl.: A61K 47/69

(54) **PRODUCTION OF SIZED MACRO- AND MICRO- ELP PARTICLES FOR DRUG DELIVERY**

(30) Priority: 05.06.2018 US 201862680828 P; 22.06.2018 US 201862688677 P
(62) Divisional of application: 19814147.5
(71) Applicant: University of Mississippi Medical Center, Jackson, MS 39216 (US)
(72) Inventor: Janorkar, Amol, Madison, 39110 (US); Cobb, Jared, Jackson, 39206 (US); Correia, John, Jackson, 39211 (US); Zai-Rose, Valeria, Flowood, 39232 (US)
(74) Representative: Schwarz & Partner Patentanwälte GmbH

(57) **Abstract**

Elastin-like polymers (ELP) are shown to demonstrate dynamic behavior atop silica, simillar to visco-elastic polymer dewetting. A combination of multiple factors are shown to contribute to this behavior including the hydrophilicity of the silica preventing the adsorption of ELP, the formation of a salt layer between ELP and silica, and the ability of the silica and salt layer to hold on to minute amounts of water for prolonged periods. Further, the addition of a polyethyleneimine (PEI) block to the terminal end of ELP allows the particle radius as well as LCST to be controlled by changing any combination of polymer concentration, NaCl concentration, and pH. The addition of the PEI block also provides the ability to crosslink the copolymers and achieve a stable particle radius after formation in harsh environments.

## Description

### CROSS-REFERENCE TO RELATED APPLICATIONS

This application claims the benefit of United States Provisional Patent Application No. 62/680,828, filed June 5, 2018, and United States Provisional Patent Application No. 62/688,677, filed June 22, 2018, and expressly incorporates herein the entirety of the foregoing applications.

### STATEMENT REGARDING FEDERALLY SPONSORED RESEARCH AND DEVELOPMENT

This invention was made with government support under Grant Number EB02006 awarded by the National Institutes of Health. The government has certain rights in the invention.

### BACKGROUND OF THE INVENTION

At the most basic level of design, the goal of a pharmaceutical agent is to target diseased tissue while preserving healthy tissue and cells. However, there are countless barriers to successfully targeting only diseased tissue in a system as complex as the human body. From macroscopic considerations of venous pathways to microscopic highly trafficked gateway of the lipid membrane, with each level of physiology specificity is crucial to reaching the desired target.

To address such issues, technology is now focusing the design of drug and gene delivery systems on nano- and micro-molecular scales. Protein and biopolymer-based carriers transport their delivery cargo through many different methods employing chemical conjugation, non-covalent association by electrostatic interactions, or physically packaging the therapeutic product.

Further, synthetic carriers are preferred over viral ones because they eliminate the risk of immunogenicity and mutagenesis, which are typically associated with viral-based systems. An optimal delivery system requires targeting specificity, efficiency in cellular and/or nuclear uptake, low cytotoxicity, and a modifiable platform to enable adaptation for different physiological environments.

An elastin-like peptide (ELP) is a genetically engineered biopolymer based on the sequence of human elastin (and therefore not recognized as foreign material by the body). It has a repeating sequence of Val-Pro-Gly-X-Gly (VPGXG), where the fourth guest residue X can be any amino acid except proline. ELP undergoes reversible inverse phase transition that occurs at a characteristic lower critical solution temperature (LCST), resulting in a change from a disordered hydrated polymer to a more structurally ordered coacervate. This LCST can be raised or lowered by modifying the ELP structure or by changing the polymer concentration and ionic strength present in solution. ELP's provision for LCST modification and its ability to impart its inverse phase transition behavior to other proteins by fusion have led to its applications in drug and peptide delivery, protein purification, and tissue engineering.

Understanding ELP's inverse phase transition behavior is important for ELP's use in applications including drug delivery, gene transfection, diagnostics, and as biologically active coatings. While the solution behavior of ELP has been extensively studied, the interaction between the ELP and a surface has not.

Innovative approaches have used biocompatible and biodegradable ELP conjugated to drugs that are less toxic than free drug systemic delivery. Further, their delivery is assisted by the enhanced permeability and retention effect due to their thermally induced coacervation. Functional or therapeutic peptides can be easily added to ELP, either by chemical reaction, genetic engineering, or both, further improving their drug delivery and function.

One previously used synthetic carrier is branched polyethyleneimine (PEI), a cationic polymer, known for its high efficiency as a non-viral vector for cell transfection. PEI's positive charges on the primary amine groups allow it to complex with negatively charged phosphates of DNA and the anionic cell membrane for efficient cell uptake. However, because transfection efficiency increases with PEI molecular weight and its cationic nature enables gene delivery, this also makes PEI cytotoxic to cells. Cytotoxicity of PEI may possibly be improved by grafting it to higher molecular weight biocompatible polymers. However, PEIs with an attached hydrophilic polymer also form less-compact structures, exhibit less efficient aggregation, and have decreased ability to evade endosomal processing. Alternatively, attached hydrophobic biocompatible moieties to PEI have shown a decrease in cytotoxicity, greater compression of carrier/DNA complexes for enhanced cell uptake, and a drastic improvement of the transfection efficiency.

Previous studies have shown that ELP-PEI copolymers exhibit both the inverse phase transition behavior of ELP as well as cell biocompatibility. ELP-PEI copolymers could serve as a modifiable platform that combines ELP's capability for thermally induced self-aggregation and biocompatibility with the transfection efficiency and control of particle radius conferred by adding a block of PEI. ELP-PEI copolymers thus have potential as a biotherapeutic delivery agent whose dosage can be controlled by cross-linking particles at the desired nanometer or macromolecular size.

Accordingly, it would be an improvement in the art to provide a method by which the size of ELP-based polymers are controlled.

### BRIEF SUMMARY OF THE INVENTION

Exemplary methods disclosed herein are designed to improve the biocompatibility and transfection efficiency of elastin-like peptide (ELP)-containing particles by controlling the size of the particles. Such a method may include adding a polymer to an ELP in solution and then coupling the polymer to the ELP to form an ELP-polymer copolymer. The method may also include heating the ELP-polymer copolymer at or above a lower critical solution temperature (LCST) of the ELP to form particles of desired size. Finally, the method may include crosslinking a plurality of ELP-polymer copolymers to form particles of the desired size resistant to disaggregation below the LCST.

In an exemplary embodiment, the method of manufacturing ELP-containing particles of controlled size includes adjusting one or more of temperature, salt, or pH of the solution of polymer and ELP to facilitate controlled particle formation of a desired size. The method can also comprise depositing the solution onto a hydrophilic surface and dehydrating the solution to form particles of a desired shape.

For example, the interaction between ELP and silica and how the ELP behaves in prolonged contact with silica above its LCST was investigated after the evaporation of water. Specifically, the distribution, size, and maturation of the ELP coacervates, after prolonged exposure to a surface above the LCST, was investigated. The de-solvated state gives the ability to image the process using a scanning electron microscope (SEM), which would not be possible if water were present. It was found that ELP undergoes a complex rearrangement where it begins as a smooth film that eventually dewets the silica to form a narrow distribution of particle sizes that dynamically grow and collapse over 24 hours.

ELP was shown to have a dynamic behavior atop silica that shows similarities to visco-elastic polymer dewetting. A combination of multiple factors was shown to contribute to this behavior, including the hydrophilicity of the silica preventing the adsorption of ELP, the formation of a salt layer between ELP and silica, and the ability of the silica and salt layer to hold on to minute amounts of water for prolonged periods. Particle size and distribution can be controlled by varying the time the ELP is raised above its LCST. This demonstrates the complex interactions that ELP can undergo when in contact with a hydrophilic surface and that ELP-surface interactions could be used to create precisely defined particles and to create surfaces that dynamically rearrange in response to their environment.

Further, the inventors discovered that adding a PEI block to the terminal end of ELP allows the particle radius as well as the LCST to be controlled by changing any combination of polymer concentration, NaCl concentration, and pH. The manipulation of the solution environment may therefore provide a method whereby ELP-containing particles can be tailored for specific applications. The addition of the PEI block also provides the ability to crosslink the copolymers and achieve a stable particle radius after formation in harsh environments.

Additional features and advantages of the disclosure will be set forth in the description which follows, and in part will be obvious from the description, or may be learned by the practice of the disclosure. The features and advantages of the disclosure may be realized and obtained by means of the instruments and combinations particularly pointed out in the appended claims. These and other features of the present disclosure will become more fully apparent from the following description and appended claims or may be learned by the practice of the disclosure as set forth hereinafter.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

In order to describe the manner in which the above recited and other advantages and features of the disclosure can be obtained, a more particular description of the disclosure briefly described above will be rendered by reference to specific embodiments thereof, which are illustrated in the appended drawings. It is appreciated that these drawings depict only typical embodiments of the disclosure and are not therefore to be considered to be limiting of its scope. The disclosure will be described and explained with additional specificity and detail through the use of the accompanying drawings in which:
Figures 1A-1C illustrate plots of Rₕ versus temperature of SynB1-ELP at concentrations of 0.8 mg/mL, 1.5 mg/mL, and 3 mg/mL, respectively, at temperatures from 20 °C to 50 °C;
Figures 2A-2C illustrate image composites at concentrations of 0.8 mg/mL, 1.5 mg/mL, and 3 mg/mL SynB1-ELP, respectively, on silica over a 24-hour incubation period;
Figure 3 illustrates plot graphs showing particle diameter versus time of SynB1-ELP at concentrations of 0.8 mg/mL, 1.5 mg/mL, and 3 mg/mL, respectively;
Figure 4 illustrates Energy Dispersive X-ray Spectroscopy of an ELP particle and the surrounding area;
Figures 5A-5D illustrate FT-IR spectra of water on silica, SynB1-ELP on silica, PBS on silica, and ELP and PBS on silica, respectively, with arrows showing decreasing water with time;
Figures 6A-6D illustrate AFM images of SynB1-ELP at time intervals of 0.5 hour, 1 hour, 1 hour, and 6 hours, respectively;
Figures 7A-7E illustrate SynB1-ELP at a concentration of 1.5 mg/mL at intervals of 0.5 hour, 1 hour, 2 hours, 2 hours, and 3.5-hours, respectively, showing formation of rims (red arrows), bands (blue arrows), and Goutte Satellites (green arrows);
Figures 8A-8D illustrate the progression of bands (blue arrows) to flat disks of SynB1-ELP at 3 mg/mL at 2 hours, 2.5 hours, 3.5 hours, and 5 hours, respectively;
Figures 9A-9D illustrate SynB1-ELP at 9 hours and 3 mg/mL with spheres, disk formation, disk fill in, and the final stage of disk formation, respectively;
Figure 10A-10D illustrate SEM images of SynB1-ELP (3 mg/mL) at 6 hours, 9 hours, 12 hours, and 24 hours, respectively;
Figure 11 illustrates the ELP-PEI synthesis using carbodiimide chemistry;
Figure 12 illustrates a graph showing FT-IR spectra of ELP, ELP-PEI 800, and ELP-PEI 10K;
Figures 13A-13B illustrate plot graphs showing OPA standard curves for ELP-PEI 800 and ELP-PEI 10K, respectively;
Figures 14A-14C illustrate plot graphs showing DLS curves for ELP-PEI 800 and ELP-PEI 10K in deionized (DI) water, 1M NaCl, and 0.2M NaCl, respectively;
Figure 15 illustrates bar graphs representing the aggregate size and transition temperature, respectively, for ELP-PEI 800;
Figure 16 illustrates bar graphs representing the aggregate size and transition temperature, respectively, for non-crosslinked neat ELP;
Figure 17 illustrates a bar graph showing the size comparisons for non-crosslinked ELP-PEI particles above Tt (top), non-crosslinked below Tt (middle), and crosslinked particles below Tt (bottom); and
Figure 18 illustrates SEM images of SynB1-ELP-doxorubicin particles at 100 um (top) and 2 um (bottom) resolution.

### DETAILED DESCRIPTION OF THE INVENTION

### I. Definitions

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which need to be independently confirmed.

Although specific terms are employed herein, they are used in a generic and descriptive sense only and not for purposes of limitation. All terms, including technical and scientific terms, as used herein, have the same meaning as commonly understood by one of ordinary skill in the art to which this invention belongs unless a term has been otherwise defined. It will be further understood that terms, such as those defined in commonly used dictionaries, should be interpreted as having a meaning as commonly understood by a person having ordinary skill in the art to which this invention belongs. It will be further understood that terms, such as those defined in commonly used dictionaries, should be interpreted as having a meaning that is consistent with their meaning in the context of the relevant art and the present disclosure. Such commonly used terms will not be interpreted in an idealized or overly formal sense unless the disclosure herein expressly so defines otherwise.

As used in the specification and claims, the singular form "a", "an" and "the" include plural references unless the context clearly dictates otherwise. For example, the term "a measurement" includes a plurality of separate measurements.

As used herein, the term "comprising" is intended to mean that the compositions or methods include the recited elements, but not excluding others.

As used herein, the term "treatment" or "treating" relate to any treatment of a disease or disorder, including but not limited to prophylactic treatment and therapeutic treatment. As such, the terms "treatment" or "treating" include, but are not limited to: preventing a disease or disorder or the development of a disease or disorder; inhibiting the progression of a disease or disorder; arresting or preventing the further development of a disease or disorder; reducing the severity of a disease or disorder; ameliorating or relieving symptoms associated with a disease or disorder; and causing a regression of a disease or disorder or one or more of the symptoms associated with a disease or disorder.

All numbers expressing quantities of ingredients, constituents, conditions, and so forth used in the specification and claims are to be understood as being modified in all instances by the term "about". Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

### II. Introduction

The present disclosure is directed to, among other things, methods for manufacturing polypeptide-based particles of controlled size. For example, the inventors have discovered that SynB1-ELP may exhibit dynamic behavior on top of silica and show similarities to visco-elastic polymer dewetting. A combination of multiple factors was shown to contribute to this behavior, including formation of a flat salt layer between ELP and silica and ability of the silica and salt layer to hold on to small amounts of water for prolonged periods. ELP particle size and distribution can be controlled by varying the time ELP spends above its LCST. Understanding of ELP-surface interactions may be used to create precisely defined ELP particles and to create surfaces that dynamically rearrange in response to their environment.

Further, exemplary embodiments are set forth employing methods and compositions for the production of novel micro- and nano-particles that combine the functionality of ELP and PEI. The inventors have discovered that by adding a PEI block to the terminal end of ELP, the particle radius as well as LCST can be controlled by changing any combination of polymer concentration, salt (NaCl) concentration, and pH.

The inventors have also observed that increasing NaCl concentration has the greatest impact on polymer behavior for ELP-PEI co-polymers, resulting in larger sizes of the particle radius. Similarly, increasing the polymer concentration may also have a demonstrated effect on both the particle radius and LCST of ELP-PEI co-polymers. Lastly, pH has a fine tuning effect on both the particle radius and LCST of ELP-PEI co-polymers. While the molecular weight of the added PEI block does not have a significant impact on radii between the two ELP-PEI copolymers, molecular weight can however, determine how responsive the copolymer will be to the small changes in solution conditions and hence its ability to be tailored for specific applications.

Therefore, implementation of the present invention allow for methods by which ELP-PEI copolymers are coarsely tuned with salt to get within a particular window of LCST and particle radius, while polymer concentration and pH are be used as modulators for fine tuning of the copolymer properties.

The details of one or more embodiments of the presently disclosed subject matter are set forth in this document. Modifications to embodiments described in this document, and other embodiments, will be evident to those of ordinary skill in the art after a study of the information provided in this document. The information provided in this document, and particularly the specific details of the described exemplary embodiments, is provided primarily for clearness of understanding, and no unnecessary limitations are to be understood therefrom.

### III. Exemplary Articles and Methods

In an exemplary embodiment of the present invention, ELP peptides may be coupled to a cargo therapeutic agent including, but not limited to, SynB1-ELP. SynB1, also known as SynB1 peptide, has the sequence RGGRLSYSRRRFSTSTGRA. In some embodiments, the ELP sequence comprises at least 5 repeats to 160 repeats of the amino acid sequence VPGXG, where X in the sequence VPGXG can be any amino acid except proline. In some embodiments, the method includes production of macro- and microparticles comprising an ELP coupled to a therapeutic agent, small molecule, or drug used to deliver an effective amount of the agent or other active molecule to a biological system, either *in vivo, in situ,* or *in vitro* for effective control or treatment. The SynB1-ELP peptide can be genetically produced from prokaryotic or eukaryotic cells using the gene encoding the SynB1-ELP peptide.

In other embodiments, the overexpressed peptide may be collected from the supernatants of expression media or cell extracts and purified using conventional purification methods known to those skilled in the art. Additionally, ELP may have the property of being thermally responsive. At or above a characteristic transition temperature (Tt), ELP can aggregate and precipitate, and when the temperature is lowered below the Tt, ELP can re-dissolve. Therefore, purification of ELP after expression in bacteria can include heating the bacterial lysate above the Tt and collecting ELP or ELP fusion proteins by centrifugation. Repeated centrifugations above and below the Tt can result in pure ELP. Purified SynB1-ELP peptide can be equilibrated into phosphate buffered saline and maintained as a stock below the LCST until use for particle formation.

In yet other embodiments, the composition further comprises a cell-penetrating peptide coupled to the ELP. Non-limiting examples of the cell-penetrating peptide are penetratin, TAT (GRKKRRQRRRPQ), SynB1, Bac, polyArg, MTS, Transportan, KLAK, or pVEC. The composition may comprise a cellular or organ targeting peptide coupled to the ELP. Non-limiting examples of cellular targeting peptide are an antibody, a receptor binding factor or substrate, or carbohydrate containing motif. Non-limiting examples of organ targeting peptide are kidney targeting peptide, a placenta targeting peptide, or a brain targeting peptide. Non-limiting examples include coupling the drug to the ELP via using a genetic sequence encoding a drug binding domain at either terminus of the ELP protein that facilitates crosslinking of the drug or biochemical compound of interest to the ELP.

Further, an exemplary embodiment of the present invention includes a method for forming micro- and macro-sized particles comprising applying solutions below the LCST containing an ELP; or ELP linked to a cargo peptide, drug, small molecule, or other polymer; or an ELP that encapsulates a non-linked peptide, drug, small molecule, or other polymer; a buffer, and a salt concentration on the hydrophillic, hydrophobic, charged, or neutral surface, such as a fused silica media. Using the surface-dewetting process, the liquid is placed into a heated environment above the LCST for a period of time until the desired macro- and micro-sized particles and shapes are obtained. Factors for particle formation may govern protein adsorption, including the expulsion of water from the protein and surface, the ability of the protein to undergo conformational changes, and the rearrangement of charges in the outer layer of the protein to align with those on the surface.

In an exemplary embodiment of the present invention, the sequence of a particular ELP can be modified such that it is possible to generate chimeras of ELP fused to therapeutic proteins or peptides or to add reactive sites for attachment of therapeutic agents. Such ELP chimeras provide certain therapeutic advantages to the therapeutic agent, such as comparatively better stability, solubility, bioavailability, half-life, persistence, and/or biological action of the therapeutic proteinaceous component or attached small molecule drug.

In another exemplary embodiment of the invention, the fused silica media can be prepared in a laminar flow hood by rinsing with acetone and then water to remove any loose or soluble debris. The silica media is soaked in 10% weight solution of hydrofluoric acid to remove any remaining contaminates. Last, the media is rinsed with water to remove residual hydrofluoric acid, rinsed with acetone, and placed in an oven until dry.

Also, in an exemplary embodiment of the invention, the aggregation of macro- and micro-ELP particles, including those coupled to therapeutic agents, such as SynB 1-ELP and like, are produced by varying the exposure of droplets on a hydrophilic surface for a period of time ranging from 0.5 hours to 24 hours at temperatures above the transition temperature. In other embodiments, the protein concentrations of the ELP or ELP-linked cargo range from 0.1 mg/mL to 10 mg/mL. The stability of the protein's structure in solution at the time of interaction with the hydrophilic surface allows for ELP aggregation. In yet, other embodiments, the formation of larger ELP aggregates using a hydrophilic surface and exposure to a heated environment above the transition temperature forms particle sizes ranging from 0.10 µm to 120 µm in size. Preferably, the particle shapes formed are spherical, hemispherical, or disc shaped. In yet other embodiments, SynB1-ELP's stability at temperatures above the phase transition, coupled with its low overall positive charge at neutral pH, prevents SynB 1-Cys-ELP from adsorbing onto the hydrophilic silica substrate.

Additionally, an exemplary embodiment of the present invention includes using one or more of polymer concentration, pH, and/or NaCl concentration to alter the LCST properties and particle radii of ELP/ELP-EPI co-polymers. In at least one exemplary embodiment, the PEI polymer sizes range from 800 g/mol or smaller to 10,000 g/mol or larger. By way of example, ELP and PEI coupled co-polymers having sizes of MW = 800 g/mol or 10,000 g/mol are referred to herein as ELP-PEI 800 and ELP-PEI 10K, respectively.

Also, another exemplary embodiment of the present invention includes a method for producing micro and macro-sized ELP and polymers particles having maximum radii ranging from 200 nm to 1700 nm and transition temperatures ranging from 24 °C to 55 °C that utilized predictable concentrations of NaCl to control aggregate sizes and to induce transition temperatures. Polymers include negatively charged, positively charged, and neutral organic polymers, as well as polypeptide polymers such as collagen and fibrinogen. Examples of organic polymers include: positively charged polymers, such as polyethyleneimine (PEI), polylysine, polyarginine, poly(diallyldimethyl ammonium chloride); negatively charged polymers such as polyacrylic acid, hyaluronic acid, poly(sodium styrene sulfonate), DNA; and neutral polymers such as polyethylene glycol, poly(vinyl pyrrolidone), poly(vinyl alcohol). Further, copolymers and amphiphilic polymers that contain fractions of two or more monomer types (positive, negative, or neutral) can be attached to the ELP.

In other embodiments, the concentration of NaCl ranges from 200 mM to 1.0 M. In yet other embodiments, increasing polymer concentration yields a higher maximum radius (Rₕ) and a slight decrease in LCST (or transition temperature (T₁)). In yet other embodiments, adjusting the pH of the ELP solution ranging from 200 mM to 1.0 M provides for fine-tuning of the aggregate size.

Another exemplary embodiment of the present invention comprises a method for producing micro and macro-sized particles containing copolymers of ELP and PEI crosslinked in a two-step process using a crosslinking agent. Other embodiments include a first step of reacting ELP with N-hydroxysuccinimide in MES buffer, pH 6.2, and EDC for 15 minutes at 4 °C. The second step includes reaction with PEI at pH 6.5, overnight at 4 °C.

In a further exemplary embodiment, a method for stabilizing the size of micro and macro-sized particles includes crosslinking the ELP-polymer particles. Crosslinking allows for the retention of the particle sizes and shapes, spherical and discs, after they are formed, while minimizing the negative environments needed to maintain them (e.g. high NaCl concentrations). In other embodiments, crosslinking is not limited and dependent upon surface properties and is achieved with any chemistry reactive towards carboxylic acids, amines, and/or thiol groups such as: multi-functional aldehydes, trans-glutaminase, epoxide chemistry, carbodiimide chemistry, isocyanate chemistry, thiol-ene/yne chemistry, acrylates, methacrylates, and so forth.

Reactions to primary amine(s) present in an ELP can be achieved via reactions using aldehydes, isocyanates, isothiocyanates, sulfonyl chlorides, ketones, nitrous acid to form nucleophiles, electrophilic substitution of the nitrogen, carbodiimide chemistry, and anhydrides. Reactions with the carboxylic acid group(s) present in an ELP can be achieved by using isocyanates, isothiocyanates, Fischer esterification, nucleophilic reactions, electrophilic reactions, non-reversible esterification, thiols, alcohol groups, primary or secondary amines, and Grignard reactions.

If cysteine residue(s) are present in an ELP, such ELPs can self-crosslinked through the cysteine-cysteine reactions after the removal of chemicals (e.g., Dithiothreitol (DTT)) that specifically prohibit such reactions. In addition, reactions to the thiol group(s) on the cysteine residue(s) present in an ELP can be achieved by using the reaction chemistries of thiol to alkene, thiol to alkynes, thiol-thiol oxidation, glutathione oxidation, thiol-gold, thiol to metals, thiol deprotonation leading to a nucleophilic reaction, Michael additions, substitution reactions using Sn2 mechanisms to form thioethers, thiol to isocyanates or isothiocyanates, thiols to acid halides, and thiols to carboxylic acids.

Reactions to the alcohol group(s) present in an ELP can be achieved by reactions with isocyanates, isothiocyanates, condensation reactions, acid halides, esterification, etherification, and nucleophilic substitutions.

The preceding chemistries used for crosslinking can also be used to attach any second type of polymer to the elastin-like polypeptide. For example, carbodiimide chemistry can be used to attach a natural or synthetic poly-amino acid such as polyarginine or polylysine to the carboxylic acid group of the ELP. It can also be used to attach synthetic, positively charged polymers such as polyethyleneimine. The primary amine group on ELP can be reacted to negatively charged polymers such as polyacrylic acid via a carbodiimide reaction. Natural or synthetic neutral polymers such as polyethylene glycol can be attached via an isocyanate reaction or through an aldehyde reaction if they are pre-functionalized with an amine group.

Purification of crosslinked particles can be performed through standard methods for small molecule removal, such as dialysis and low speed centrifugation.

In at least one embodiment, the ELP or ELP-PEI copolymer micro and macro-sized particles are dissolved in water, and the appropriate environmental conditions are adjusted to achieve the desired radius described in the previous examples. ELP or ELP-PEI copolymers particles may also be heated above the transition temperature in an oven, and 10-fold concentration of glutaraldehyde may be added to the solution. The mixture may be reacted for 1 hour and, afterwards, particles may be purified by utilizing the inverse transition temperature of the ELP.

The present invention relates also to the following embodiments, without being restricted thereto.

Preferred embodiments:
1. A method of manufacturing polypeptide-based particles of controlled size, comprising:
   adding a polymer to an elastin-like polypeptide (ELP) in a solution;
   coupling the polymer to the ELP to form an ELP-polymer copolymer;
   heating the ELP-polymer copolymer at or above a lower critical solution temperature (LCST) of the ELP to form particles of desired size; and
   crosslinking a plurality of ELP-polymer copolymers to form particles of the desired size resistant to disaggregation below the LCST.
2. The method of embodiment 1, wherein the ELP comprises about 5 to about 320 repeating units of the amino acid sequence VPGXG, where X is any amino acid except proline.
3. The method of embodiment 2, wherein X is valine.
4. The method of embodiment 1, wherein the polymer includes functional groups selected from carboxylic acid, carboxylate, amine, hydroxyl, and thiol groups.
5. The method of embodiment 1, further comprising adjusting the polymer concentration, salt concentration and pH of the solution to facilitate particle formation of a desired size.
6. The method of embodiment 1, wherein cross-linking comprises reacting the ELP-polymer copolymer with a cross-linking agent.
7. The method of embodiment 6, wherein the cross-linking agent includes functional groups selected from aldehydes, isocyanates, isothiocyanates, acid halides, and ketones.
8. The method of embodiment 1, wherein cross-linking comprises oxidizing thiol groups between cysteine residues to forming sulfide bonds.
9. The method of embodiment 1, further comprising coupling either genetically or chemically a therapeutic agent to the polypeptide-based particles.
10. The method of embodiment 9, wherein the therapeutic agent is selected from a drug, a gene, a protein, and a peptide.
11. The method of embodiment 9, wherein the therapeutic agent is coupled to the polypeptide-based particles using a drug binding domain.
12. The method of embodiment 1, further comprising raising the concentration of the polymer in the solution to increase particle size.
13. The method of embodiment 1, wherein the copolymer is a peptide or an organic polymer.
14. The method of embodiment 13, wherein the organic polymer is acidic, basic or neutral.
15. The method of embodiment 1, wherein the desired particle size is selected from nanoparticle, microparticle, and macroparticle.
16. The method of embodiment 1, further comprising raising the salt concentration in the solution to between 0.1 M NaCl to 1.0 M NaCl above the LCST to increase particle size.
17. The method of embodiment 1, further comprising adjusting the pH of the solution to between pH 3 and pH 10.
18. The method of embodiment 1, wherein the polymer is coupled to an amino terminus or a carboxylic acid of the ELP.
19. A method of manufacturing polypeptide-based particles of controlled size and shape, comprising:
   adding a polymer to an elastin-like polypeptide (ELP) in solution;
   coupling the polymer to the ELP to form an ELP-polymer copolymer;
   adjusting temperature, salt, and pH of the solution to facilitate particle formation of a desired size;
   depositing the solution on to a hydrophilic surface;
   dehydrating the solution to form particles of a desired shape; and
   cross-linking a plurality of ELP-polymer copolymers to form particles of the desired size resistant to disaggregation below LCST.
20. The method of embodiment 19, where the hydrophilic surface is silica.

### EXAMPLES

### Example 1: Production of Surface Aggregated ELP Cargo Peptide Particles.

The ELP-cargo peptide selected for the macro- and micro particle formation was a SynB 1-Cys-ELP peptide construct. SynB1-Cys-ELP peptide was prepared from a plasmid containing a repeat structure of MRFFRLSYSRRRFSTSTGRCGPG (VPG[V₅G₃A₂]G)₁₅₀WP encoding a peptide of MW = 61,739 daltons. The plasmid was expressed by cloning into a pET25b vector and transformed into BLR (DE3) *Escherichia coli.* The overexpressed polypeptide was collected from the transformation media and purified by four rounds of thermo-cycling to a translucent pellet. Samples were equilibrated into PBS (phosphate buffered saline (150 mM NaCl, 2.7 mM KCl, 20 mM Na₂HPO₄, 1.8 mM KH₂PO₄)), by dialysis using a Slide-A-Lyse MINI Dialysis device purchased from Thermoscientifc with a 3,500 molecular weight cut-off.

Fused silica media selected as the substrate for ELP-Cargo Peptide particle deposition were 1.5-inch diameter disks obtained from Thorlabs. Media disks were cleaned by a standard cleaning procedure for fused silica in a laminar flow hood entailing first rinsing the substrates with acetone and then water to remove any loose or soluble debris. The disks were soaking in a 10% weight per volume solution of hydrofluoric acid for 5 minutes to remove any remaining contaminates. Afterward, the substrates were rinsed repeatedly with water to remove any acid, rinsed with acetone, and dried in an oven at 50 °C for 12 hours.

In preparation of ELP-Cargo Peptide particle formation, a stock of SynB1-Cys-ELP stored in PBS was used to make three increasing concentrations of SynB1-Cys-ELP: 0.8 mg/mL, 1.5 mg/mL, and 3 mg/mL. A 10 µL aliquot of SynB1-Cys-ELP solution was placed as a droplet onto a fused silica disk. The silica disk was subsequently placed into a 60-mm cell culture plate to prevent any accidental contamination from dust and incubated in a Thermo Lindberg/ Blue M digital oven (Thermo Scientific, Waltham, MA) set at 50 °C. Samples of the droplets at different time points were analyzed using different measurements over a 24-hour incubation period to visualize the surface mediated ELP behavior.

### Example 2: Methods of Analysis of Surface Aggregated ELP Cargo Peptide Particles.

Several measurement methods were used to evaluate the properties of the surface aggregated ELP Cargo particle formed that included electron microscopy, chemical composition, light scattering, atomic force microscopy, and FT-IR spectroscopy. The first analytical measurement used was Dynamic Light Scattering (DLS) as an approach to study the size of the coacervates as they partition between the two phases. Though limited by the size of particles it can measure, DLS is able to show the formation of coacervated droplets with radii of hydration (Rₕ) up to several µm. Dynamic Light Scattering was performed using a DynoPro NanoStar instrument (Wyatt Technology, Santa Barbara, CA) equipped with Dynamics V7 version 7.19 software. SynB1-Cys-ELP droplet samples corresponding to concentrations of 0.8 mg/mL, 1.5 mg/mL, and 3 mg/mL were centrifuged for 3 min at 5 °C at 50,000 rpm in a Beckman Optima TLX ultracentrifuge (Palo Alto, CA). A volume of 10 µL sample was loaded directly from the centrifuge tube into a DLS cuvette and allowed to equilibrate for 15 min inside the instrument. The experiments were conducted with a ramp rate of 0.20 °C /min from 20 to 50 °C.

A visual method employed a Scanning Electron Microscope (SEM) measurement of particle size using a Leica EM ACE600 coating system (Germany) that vented with argon was used to sputter-coat the samples with a 4-nm thick gold-palladium coating to prevent charge buildup when imaging. A Zeiss Supra 40 electron microscope (Germany) using an SE2 Everhart-Thornley detector, an aperture size of 30 µm, and an accelerating voltage of 3 keV was used for imaging the samples. SEM Image Analysis for particle size analysis was obtained using ImageJ version 1.51k (NIH). A calibrated scale was based upon measuring the scale bar on the SEM image by the ImageJ software, after which the particle diameters were measured by using the elliptical tool to outline individual particles. The minimum sample size of 50 particles established an accurate representation of the population. The particle analysis was reported as the mean diameter ± 95% confidence interval.

A chemical composition of samples was mapped using Energy Dispersive X-ray Spectroscopy (EDS) from EDAX Octane Plus (AMETEK Mahwah, NJ) that was coupled to the SEM described above. The accelerating voltage of the EDS was set to 5 keV for EDS to maximize the number of chemical elements that were detected and to minimize sample charging. EDAX Team software version 6.43 was used to collect and analyze the data.

Atomic Force Microscopy images were obtained using a Bruker Bioscope Catalyst AFM (Billerica, MA). Select samples were imaged in the large amplitude Scanasyst^{®} mode with a Scanasyst-air probe at a rate of 0.250 Hz. Images were analyzed using Gwyddion software version 2.64.

Attenuated Total Reflectance (ATR) Fourier Transform-Infrared (FT-IR) Spectroscopy was performed using Perkins-Elmer Spectrum 100 FT-IR spectrometer (Waltham, MA) with a universal attenuated total reflectance accessory equipped with a diamond crystal and a fixed beam incidence angle. Independent samples were prepared by placing a volume of 10 µL at 4 °C onto silica disks at room temperature and subsequently incubating them at 50 °C. The samples were placed into a portable desiccator for transport to minimize adsorption of atmospheric water and were placed onto the ATR crystal without any additional preparation. All spectra were collected and analyzed using Spectrum software version 6.0.2. A background spectrum was taken to eliminate the presence of noise caused by moisture and carbon dioxide from the background environment. The samples were then placed on the crystal and secured via a pressure clamp at 110 psi. Sixteen scans were taken in the spectral range of 4000 cm⁻¹ to 650 cm⁻¹ with a spectral resolution of 4 cm⁻¹. The scans were plotted as transmission (%) versus wavenumber (cm⁻¹).

### Example 3: Analysis of the Properties of Aggregated ELP Therapeutic Agent Particles on a Silica Surface.

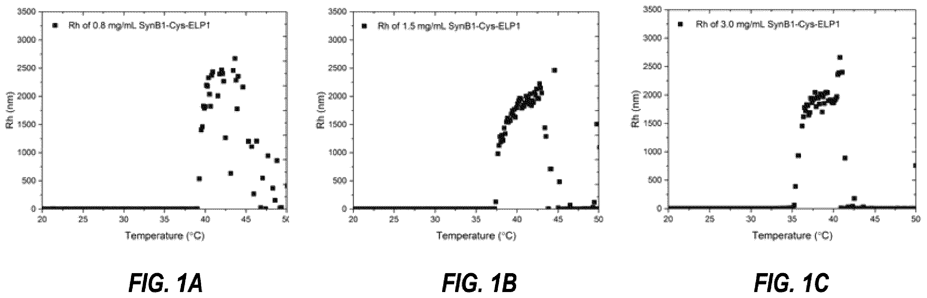

DLS measurements showed that the three concentrations of ELP exhibited a concentration dependent LCST from 36 to 39 °C. The DLS data of the three concentrations showed that coacervates of increasing Rₕ began to form above the corresponding LCST (Figures 1A-1C). A drop in the Rₕ values starting around 40 to 45 °C was observed for all concentrations due to sedimentation of large 2.0 - 2.5 µm coacervates to the bottom of the DLS container. The DLS measurements guided the selection of temperatures above LCST at which SEM experiments were performed to capture the sedimented coacervates forming contacts with the silica surface. The SEM experiments were performed at 50 °C, approximately 10 to 14 °C higher than the LCST to capture stable coacervates at all three concentrations.

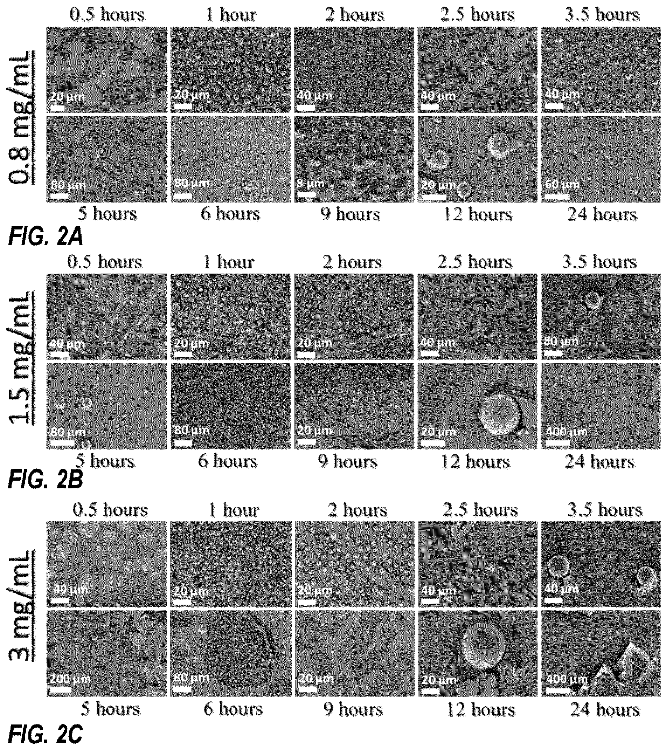

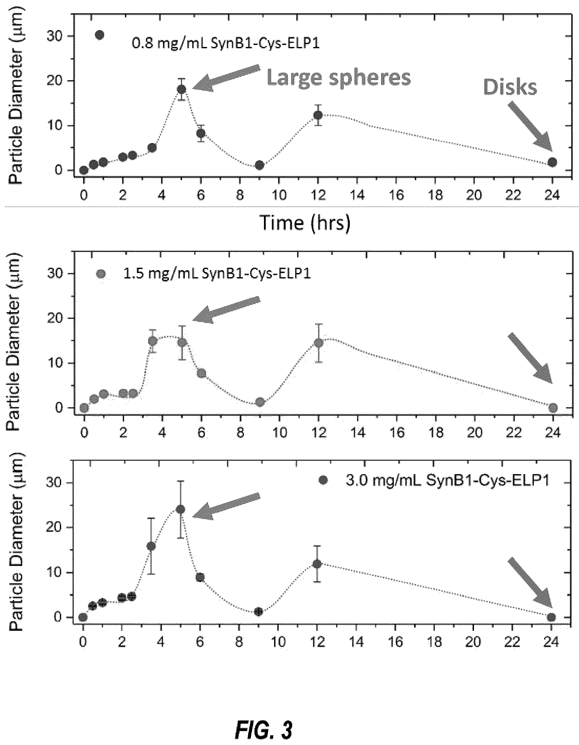

The SEM analysis of SynB1-ELP coacervate formation on silica surface was performed to examine the time dependent interaction of ELP upon prolonged contact with silica above its LCST. It was observed that after de-solvation, upon contact with silica media, the ELP formed into large spherical particles (denoted by arrows) between 2 and 6 hours that dynamically grew and collapsed over time and grew again into larger particles (Figures 2A-2C and Figure 3). In Figures 2A-2C, time-lapse image composites of the concentrations ELP on silica media were investigated at 0.8 mg/mL, 1.5 mg/mL, and 3 mg/mL, respectively. All three concentrations showed a similar pattern where at 0.5 hours the droplets retracted from the silica to expose a layer of salt. Eventually, at the 1-hour time point the ELP spontaneously dewetted the substrate to form very uniform distributions of particles that continually grew up to 5 hours. From 6 to 9 hours the particles rapidly decreased in size, until at 12 hours they became large again. At 24 hours the particles had collapsed into smaller sized particles that were disk shaped (denoted by arrows in Figure 3)r. Images were displayed at different magnifications to illustrate the prominent features.

In Figure 3, graphs depict the particle diameters measured from the SEM images: 0.8 mg/mL, 1.5 mg/mL, and 3 mg/mL, respectively. The graphs illustrate the narrow particle distribution and growth from 0.5 to 2.5 hours, which was followed by a rapid increase in size from 3.5 to 5 hours. From 6 to 9 hours the particle size deteriorated, but rebounded to a much larger value at 12 hours. The water content of PBS containing particles decreased in a log scale from 32% at one hour to 5% by 12 hours upon drying.

As a general trend, all three concentrations (0.8, 1.5, and 3 mg/mL, respectively) exhibited a growth in mean particle diameter from 0.5 hours (1.26 ± 0.07 µm, 2.00 ± 0.01 µm, 2.55 ± 0.13 µm) to 2 hours (2.87 ± 0.82 µm, 3.25 ± 0.36 µm, 4.36 ± 0.44 µm). The diameters leveled off at 2.5 hours for all three concentrations (3.29 ± 0.77 µm, 3.23 ± 0.39 µm, 4.68 ± 0.43 µm). In between, the 1.5 and 3 mg/mL samples drastically increased in size at 3.5 hours (14.89 ± 2.50 µm, 15.88 ± 6.20 µm). By the 5-hour time point all three concentrations showed a large mean particle diameter (18.11 ± 2.4 µm, 14.54 ± 3.80 µm, 24.04 ± 6.40 µm). After 6 hours (8.24 ± 1.90 µm, 7.73 ± 0.58 µm, 8.89 ± 0.81 µm), the mean diameters dropped until 9 hours to their lowest measured values (1.10 ± 0.16 µm, 1.29 ± 0.17 µm, 1.27 ± 0.13 µm). The particle diameters surged to higher values at 12 hours (12.34 ± 2.30 µm, 14.46 ± 4.30 µm, 11.91 ± 4.04 µm) and by 24 hours the 1.5 and 3 mg/mL sample particles collapsed onto the surface of the substrate and form disks, while the 0.8 mg/mL sample continued to show particles with a small mean diameter of 1.78 ± 0.26 µm.

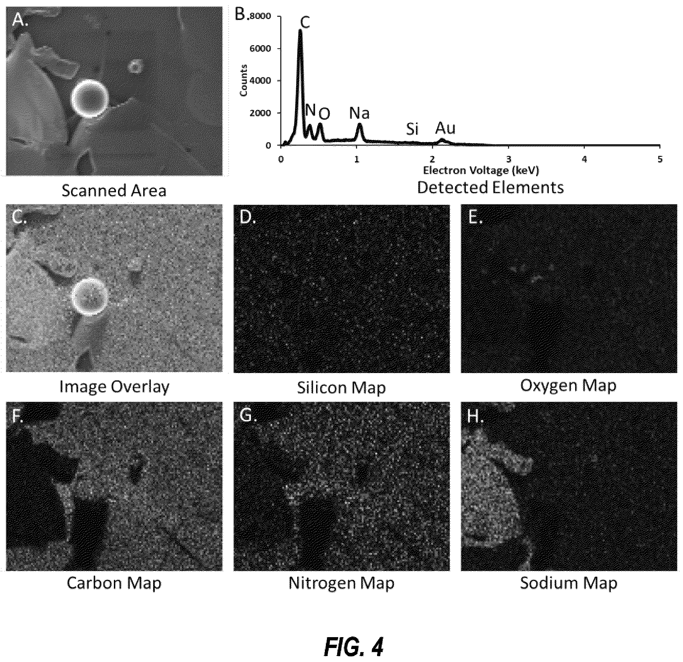

EDS imaging was used to map the elements present on the sample surface, specifically to identify the chemical composition of the larger raised surface structures in Figures 2A-2C and to confirm that the spherical structures observed were organically based ELP and not crystallized salt. In Figure 4, the scanned area shows the original image analyzed (A). The detected elements are shown in the EDS spectrum (B). In (C), the image overlay shows all elements superimposed on the original image where pink (D) represents silicon, red (E) is oxygen, gray (F) is carbon, yellow (G) is nitrogen, and cyan (H) represents sodium. EDS confirmed the presence of all six elements: carbon, nitrogen, oxygen, sodium, silicon, and gold. Gold is from the sputter coated Au/Pd film that was used to prevent charging. Carbon and nitrogen were shown to be present over the entirety of the surface except in the larger raised crystal structures (F & G). Sodium was found over the entire surface with the highest prevalence in the raised structures confirming them to be salt (H). Oxygen and silicon were found to be evenly distributed over the entire sample surface (D & E). This is not surprising since the substrate was composed of silicon and oxygen. Oxygen was also present in the other components of the sample such as the sodium phosphate in the PBS and along the backbone of the ELP.

SEM images showed a crystalline NaCl layer had formed at the 0.5-hour time point (Figures 2A-2C), and SEM-EDS confirmed the crystal structures contained sodium (Figure 4). While, this indicated that the NaCl film in combination with the silica might play a role in the formation of the observed structures, the role that the individual NaCl and silica components played in the dynamic change of ELP on the surface was unclear. To determine the formation of the larger ELP structures and their subsequent collapse and regrowth, FT-IR spectroscopy was used to measure how long water was present in the ELP structures during the drying process. FT-IR showed very strong absorption bands for water at 1643, 2127, and 3400 cm⁻¹ and used to detect very small quantities of water present in the sample. The spectra of dry silica and water on silica were used as the baseline measurements in the absence of water and the maximum amount of water on silica. Water retention of silica was examined as the first step.

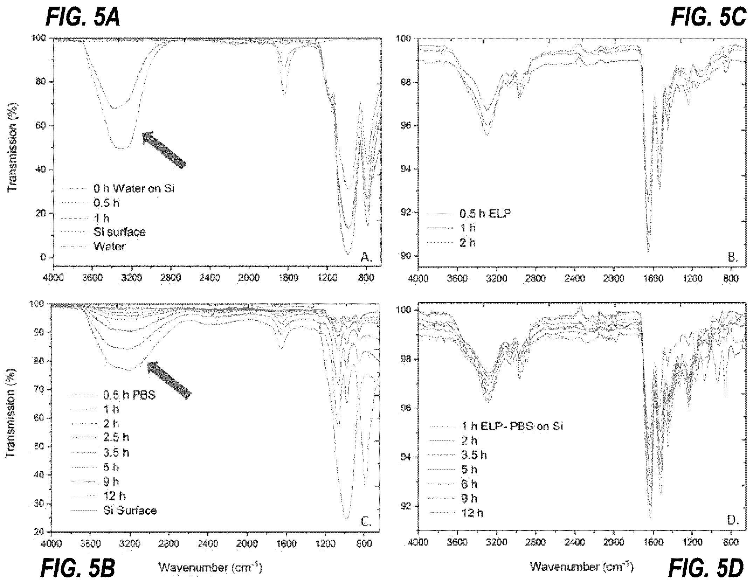

In Figure 5A, after 30 minutes of incubation at 50 °C, only a very small amount of water remained bound to the silica, as indicated by the water peak at 3300 cm⁻¹, and by the 1-hour time point little to no water remains. This confirmed the hydrophilicity of the silica and showed its ability to bind water to its surface. FT-IR spectra taken of the ELP solution in DI water dried on the silica indicated that at the 0.5-hour time point no detectable amounts of water were present, as shown in Figure 5B. Additional ELP drying times at 1 and 2 hours showed no change in spectral peak shifts or intensities and confirm the results at 0.5 hours. On the other hand, PBS showed one of the characteristic peaks of water in the spectra at 3300 cm⁻¹ (Figure 5C). Similarly, a strong peak for water at 3300 cm⁻¹ was seen at the 0.5-hour time point and slowly decreased in intensity all the way to 12 hours. The strong affinity of the various salts present in PBS for water coupled with the hydrophilicity of the silica substrate explained the dynamic rearrangement of the ELP on the surface. FT-IR was also used to confirm that ELP was present atop the silica surface during the entire incubation process (Figure 5D).

Atomic Force Microscopy (AFM) is the preferred method for imaging the coacervate particles because of its ability to obtain high magnification images in a fluid environment at various temperatures using a heated stage. Solution-based AFM is only useful after the coacervates have settled on the surface of the container because the size of the coacervates floating in solution are too large and scatter the laser light used to image them. Therefore, while SEM is useful at imaging objects, ultimately the technique can only provide two-dimensional images of three-dimensional objects and offers no way to quantify whether an object is truly raised off the surface.

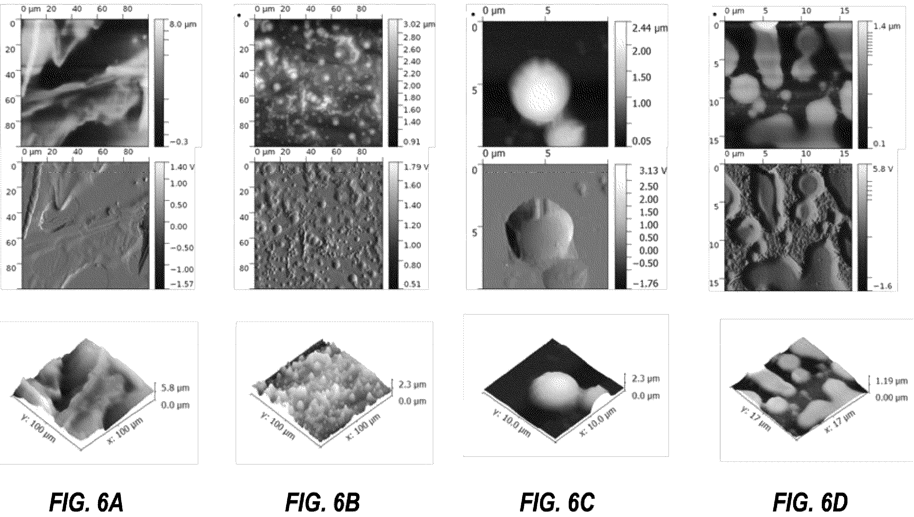

In Figure 6A, AFM confirmed that small rim structures were present at the 0.5-hour time point, however they were raised off the surface. A larger scan area at the 1-hour time point (Figure 6B) confirmed that the spheroidal objects were protruding from the surface and not concave ellipses. At 1-hour, larger particles were observed to be formed as depicted in Figure 6C of an individual particle. By the 6-hour time point, particles were interconnected and flat in appearance (Figure 6D).

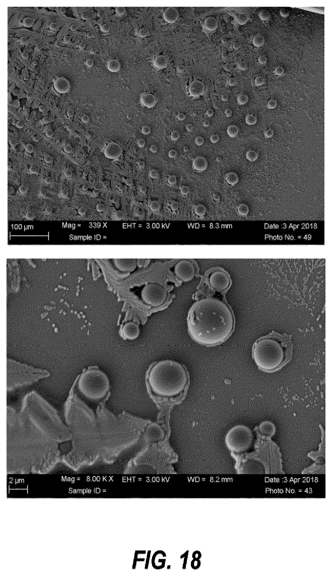

In other examples, as a proof-of-principal, SynB1-ELP was conjugated to doxorubicin for cancer treatment using a cleavable linker having a cysteine residue coupled to doxorubicin via a disulfide bond, as described in U.S. Patent No. 8,252,740 (incorporated by reference). Figure 18 illustrates SEM images of SynB1-ELP-doxorubicin particles at 100 um (top) and 2um (bottom) resolution. As shown in Figure 18, SynB1-ELP-doxorubicin particles were spherical and identical in size to unconjugated SynB 1-ELP particles.

### Example 4: Physical Behavior of ELP-Therapeutic Agents on Silica Substrate.

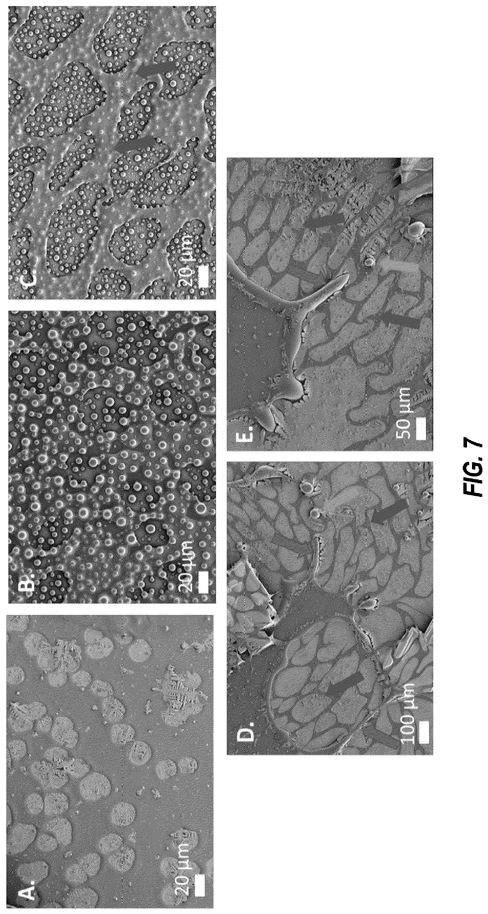

The ELP solution was placed onto the substrate at 4 °C, a temperature where the ELP was still hydrated by the surrounding water, and the individual protein molecules remained isolated from each other, as can be seen in the DLS results (Figures 1A-1C). All three concentrations ((0.8, 1.5, and 3 mg/mL, respectively) exhibited a similar behavior after the evaporation of water at 0.5 hours up to the 3.5-hour time point. A rough opaque film was observed on the substrate when incubated at 50 °C for 0.5 hours, with several large NaCl structures present around the outer diameter of the film. Upon closer observation with SEM, an ELP coating appears to have formed on top of a thin layer of crystalline NaCl that precipitated earlier. SEM-EDS confirmed the presence of sodium evenly distributed on the surface of the sample (Figure 4). The ELP film appears to be retracting as indicated by ellipse shaped holes in the film exposing both a thin layer of NaCl and some of the substrate by the 1.5 mg/mL sample as shown in Figure 7A. On top of the ELP film and scattered on the surface of the sample, many small spherical ELP occlusions were observed, which is consistent with the DLS measurements and from SEM observations.

As the sample was elevated in temperature, the ELP was forced to coalesce above its LCST as the coacervates form, and they sedimented to the bottom of the droplet, where they destabilized as they encounter the substrate. However, because the surface of the silica substrate was hydrophilic in nature, it retained the water molecules bound at the interface as indicated by FT-IR (Figure 5A) and disallowed the ELP particles to adsorb. Unable to re-solvate, the coacervates came together near the surface of the substrate to form a large coalesced globule. It is suggested that that as the ELP coacervates contacted the surface, they could no longer maintain the surface tension necessary to keep their form. After a certain amount of water had evaporated from the sample, the NaCl concentration reached a critical point where it underwent heterogeneous nucleation at the interface of the silica forming a thin layer of salt. The shrinking water interface began to force the ELP globule to interact with the NaCl layer due to the increased hydrostatic pressure forcing it downward to form a coating on the salt. Further support is drawn from the SEM images at 0.5 hours that show a coating of ELP retracting away from an undercoating of salt. During this process, a low number of smaller ELP particles had formed and were deposited on the surface of the ELP coating as observed in the SEM pictures at 0.5 hours for all three concentrations (Figures 2A-2C).

The 0.5-hour time point shows that ELP began to contract together exposing the NaCl layer. By doing so it created a void in the film as the ELP begin to accumulate at the outer edge of the holes near the exposed salt. This is also a feature exhibited by polymers in a metastable state, typically during an annealing process where they are below their glass transition temperature (T_{g}). This metastable state allows for partial wetting of the substrate without a spontaneous self-association of the polymer. If annealing of a metastable polymer continues above its T_{g}, it exhibits spontaneous dewetting of the substrate and forms droplets. While T_{g} is not known to occur in ELP, a similar phenomenon is seen at the 1-hour time point for all three concentrations (Figures 2A-2C, 7B). SEM shows that the majority of the ELP spontaneously retracted above the NaCl layer forming very uniform spherical coacervates. The remaining ELP contracted inward leaving behind rim structures. By the 2-hour time point, all three concentrations show that more droplets formed while others coalesced into larger ones and the rims retract into distinct structures (Figures 7C-7E).

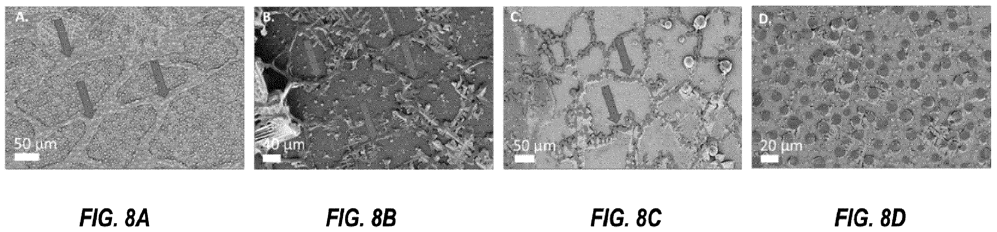

At 3.5 hours, larger particles attached to the protein bands started to form for the 1.5 and 3 mg/mL samples. The formation of these larger particles is attributed to coalescence of the rim structures due to Plateau-Raleigh instability as the rim retracted inward. This instability occurs from a liquid's innate ability to minimize its surface area when in contact with a surface that it dislikes. The images indicate the Raleigh instability incurred by the ELP is characteristic to that of a viscoelastic polymeric solution due to the formation of rim structures and smaller size Goutte satellite droplets that are present between the rims and coacervates (Figures 7D-7E). The 5-hour time point showed smaller band structures that did not coacervate and contract into uniform flat disks as demostrated by the 3 mg/mL sample (Figures 8A-8D).

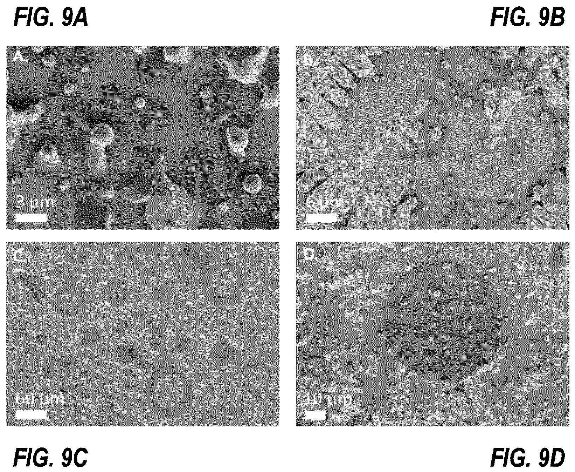

At the 6 hours-time point, the ELP again spontaneously retracted from the surface for all three concentrations, forming numerous small non-uniform particles. The larger coacervates observed at the 3.5 and 5-hour time points began to destabilize and collapse downward onto themselves (Figures 2A-2C). At 9 hours, the sample particles destabilized and began to flow outward (Figure 9A). The polypeptides formed rings on the surface (Figure 9B) that eventually filled themselves in with more ELP (Figures 9C-9D).

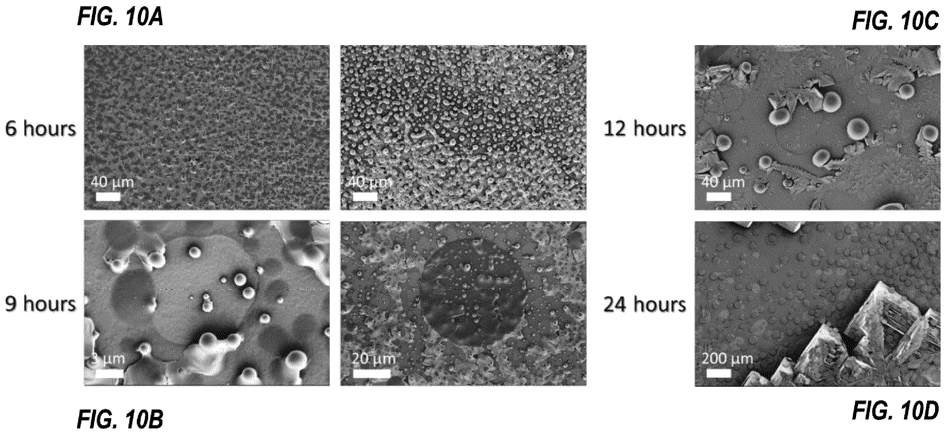

The 6-hour time point ends particle growth for all three sample concentrations and a combination of collapsed structures formed a film. In Figure 10A, at 6 hours, the ELP film (Left) retracted from the surface (Right). Figure 10B shows that at the 9-hour time point, the dissolution of the spherical particles (Left) and the formation of large circles on the sample surface (Right) occurred. Figure 10C shows the 12-hour time point large spheres; and finally, and Figure 10D shows that at the 24-hour time point, the spheres were collapsed. Between the 5 and 6-hour time points the spherical structures collapsed forming a coating on the NaCl layer. With time, the release of water from the NaCl layer got trapped between the NaCl and ELP and built a water layer at the interface eventually causing the ELP to contract upward entrapping water in the core of the spheres. By the 9-hour time point the spheres for all three concentrations destabilized and ELP-water mixtures aggregated to form disks (Figure 10B). As the water evaporated from the ELP disks the concentration of the polymer increased and, as seen at the 12-hour, the ELP underwent a dynamic rearrangement to minimize its contact with the substrate (Figure 10C). By the 12-hour time point, large hemispherical objects were protruding from the surface of all three sample concentrations, shown Figures 2A-2C and Figure 10C. These structures are similar to those observed at the 3.5 and 5-hour time points. These structures are connected by smaller string structures, but are more abundant, and appear to be flat on the bottom. At 24 hours, the higher concentrations of 1.5 and 3 mg/mL show numerous large disks that could have formed by the collapse of larger spheres (Figures 2B-2C). The lowest concentration exhibits spherical structures like what was observed at the 9-hour time point (Figure 2C).

### Example 5: Copolymer ELP-PEI Synthesis and Analysis.

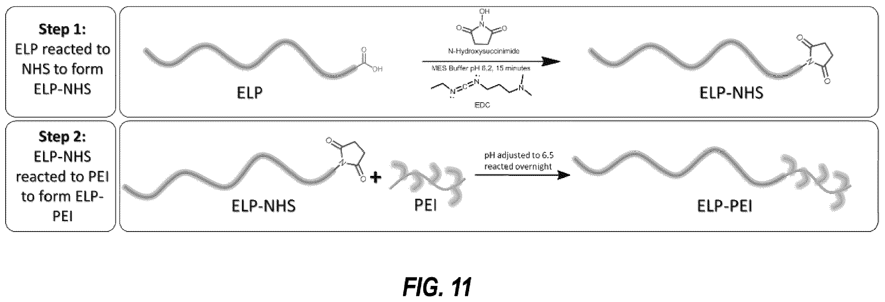

ELP (MW = 17,000 Da), produced from genetically engineered *E. coli,* was coupled to PEI *(Polysciences)* having sizes of MW = 800 g/mol or 10,000 g/mol in a two-step reaction, as shown in Figure 11. In the first step, the ELP was reacted with N-hydroxysuccinimide (10:1 molar ratio NHS to ELP). The reaction was accomplished using 1-ethyl-3-(3-dimethylaminopropyl) carbodiimide (EDC) as a catalyst (10:1 molar ratio EDC to ELP) in MES buffer (10 mg/mL ELP in MES buffer) at a pH of 6.2 for 15 minutes. PEI (10:1 molar ratio PEIto ELP) was added to a separate container and dissolved in MES buffer and subsequently titrated to a pH of 6.5. The PEI solution was cooled to 4 °C, and the ELP solution was added. This reaction was allowed to proceed overnight. Purification and removal of unreacted PEI were achieved by inverse phase transition cycling. The resulting copolymers are referred to as ELP-PEI 800 and ELP-PEI 10K.

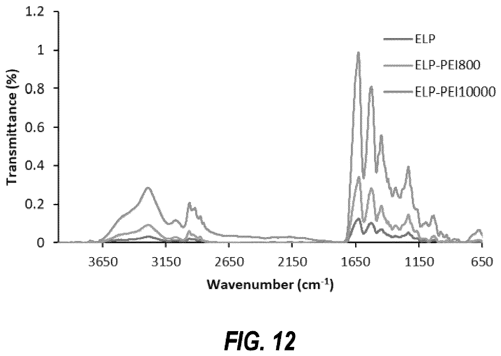

Based on preliminary studies with ELP-PEI 10K coatings for the culturing of pre-adipocytes, a ratio of 1.2:98.8 ELP-PEI 10K to ELP was selected for testing. An equivalent weight percent of ELP-PEI 800 (15:85 ELP-PEI 800 to ELP) to that of ELP-PEI 10K was used to observe the difference the polymers exhibited given the selected environment. Because the regular repeat structure (NH₂-[CH₂]) of PEI and the large amount of amide groups present in ELP, it was very difficult to distinguish ELP-PEI 800 and ELP-PEI 10K copolymers with traditional techniques such as FT-IR, as shown in Figure 12. Fourier Transform-Infrared Spectroscopy (FT-IR) was performed using a Perkins-Elmer Spectrum 100 FT-IR equipped with an attenuated total reflectance (ATR) attachment to characterize the structure of the copolymer.

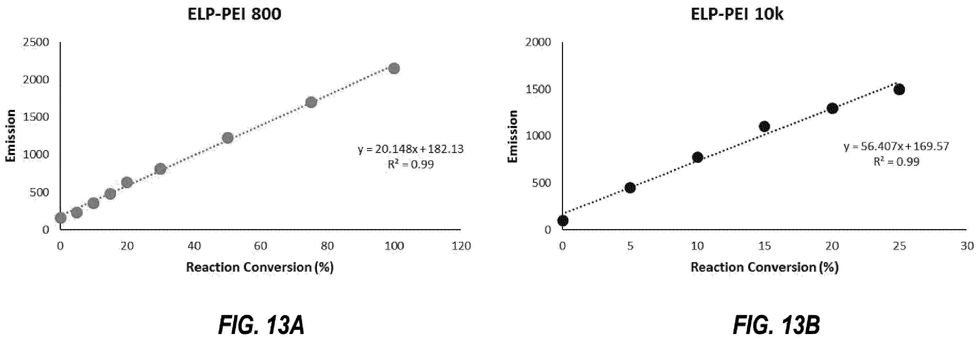

A new protocol was developed to measure the efficiency of the reaction using an O-phthlaladehyde (OPA) assay system. The reaction conversion was determined by labeling the copolymer with OPA *(Thermo Sci),* after which the fluorescence was measured and compared to a previously generated standard curve (generated by first measuring the fluorescence of different combinations of ELP to non-reacted PEI (1:0, 1:0.05, 1:0.10, 1: 0.15, etc.) at five concentrations (0.1, 0.2, 0.3, 0.4, 0.5 mg/mL)). The protocol showed that while the increased amination due to PEI conjugation increased the OPA fluorescence, the PEI fluorescence was somewhat masked by ELP at lower percent conjugations. This is an important finding that helps to more accurately estimate an unknown percent conjugation. However, as shown in Figures 13A-13B, the amount of fluorescence that ELP-PEI 800 and ELP-PEI 10K copolymer molecules emitted at different percent conjugations had a linear correlation to OPA fluorescence present; in both cases the R² = 0.99.

A Dynapro Nanostar DLS instrument *(Wyatt Tech)* was used to determine aggregation sizes of ELP-PEI 800 and ELP-PEI 10K copolymers in aqueous solutions by altering their concentration (0.1, 0.17, 0.3 mg/mL), solution pH (3, 7, 10), and NaCl concentration (0, 0.2, 1 M NaCl). For crosslinked particles, they were incubated at 4 °C for 1 hour and subsequently DLS was performed at a temperature below the transition temperature at 20 °C (See below).

### Example 6: ELP-PEI Transition Temperature Properties at Different Salt Concentrations.

The following studies were performed is determine the ELP phase transition properties when fused to two molecular weights of PEI, 800 g/mol and10,000 g/mol, and the influence on the coacervate size by environmental factors including PEI concentration, pH, and NaCl concentration.

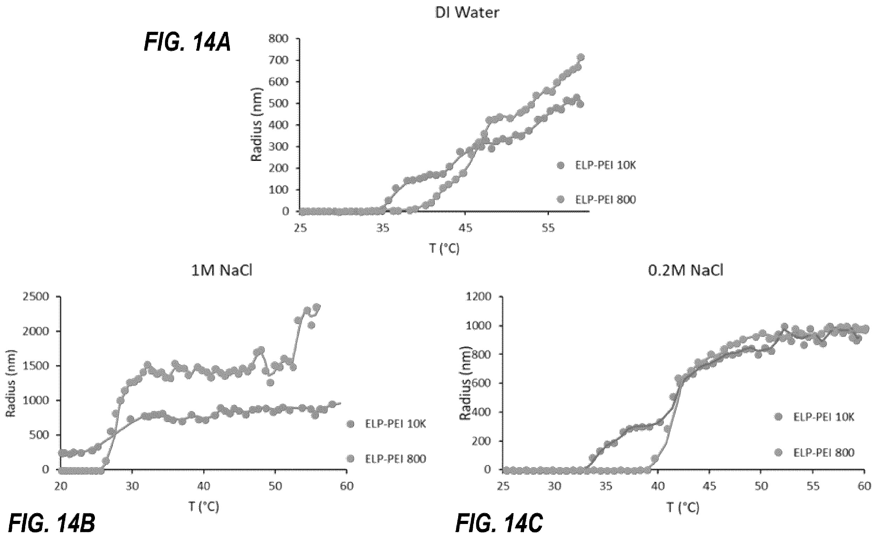

In the absence of NaCl, DLS measurements on ELP-PEI 800 showed a multiple modal transition curve, as shown in Figure 14A. In contrast, in both 0.2 M and 1 M NaCl solutions (as shown in Figures 14B-4C), indicating that the ELP-PEI 800 exhibits similar transition temperature properties as neat ELP.

Similarly, in absence of NaCl ELP-PEI 10K exhibited a more complex, tri-modal transition curve with on onset of 35 °C and a radius of hydration (Rₕ) value of 550 nm (Figure 14A). However, in 0.2M NaCl, the transition curve shifts to bimodal with an onset of 33 °C and Rₕ value of 950 nm (Figure 14C). At a higher NaCl concentration of 1M, the curve exhibits a single transition at 23 °C with the Rₕ value of 950 nm (Figure 14B).

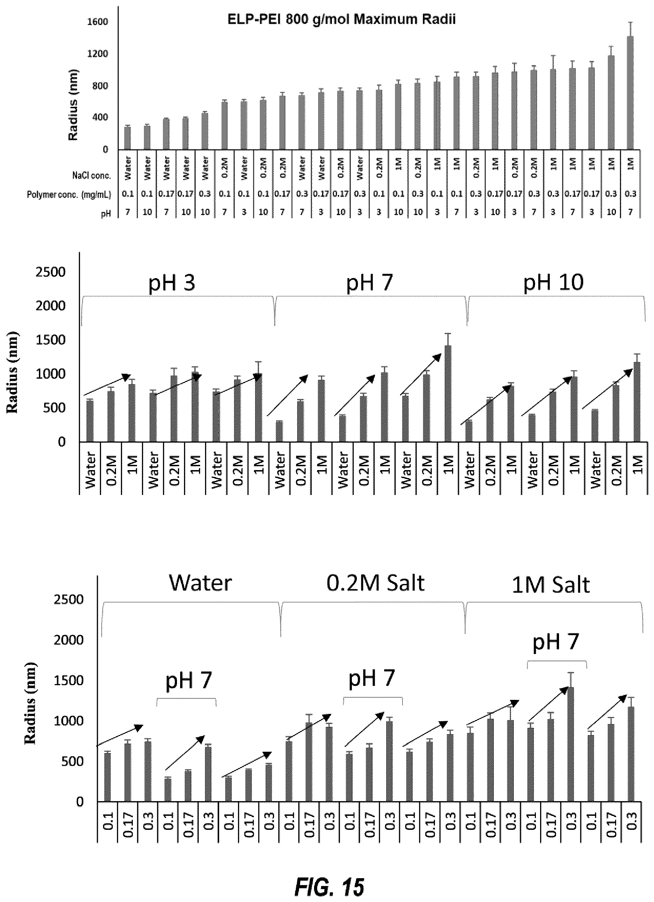

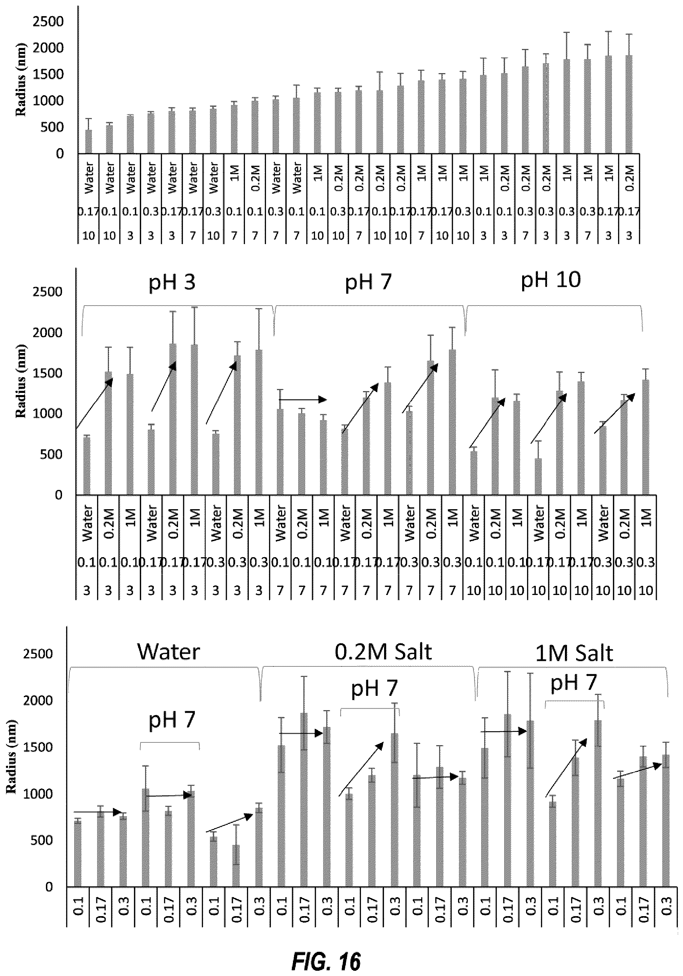

In the next set of studies, the effect of NaCl concentration on the Rₕ of ELP-PEI 800, and neat ELP as shown in Figure 15 and Figure 16, respectively. An increase in the salt concentration yielded a higher maximum radius (Rₕ) in ELP-PEI 800 of solutions from pH 3 to pH 10. A similar effect of salt concentration was observed for ELP-PEI 10K. In contrast, for neat ELP only 0.2M NaCl increased particle size which may in part due to the lack of the additional charges provided by the polymer. An increase in polymer concentration, either ELP-PEI 800 or 10K, regardless of salt concentration or pH yielded a higher maximum radius (Rₕ) and a slight decrease in LCST due to increased hydrophobic interactions forming larger, more stable aggregates. A similar effect of polymer concentration was observed for ELP-PEI 10K. In contrast, for neat ELP only at pH 7 in the presence of NaCl did aggregates form larger sized particles. Note, for each triplet (0.1, 0.17. and 0.3) under water or salt, the left grouping is pH 3, middle is pH 7 and right is pH 10. Other results suggested that the polarization of the water molecules decreases hydration of the ELP amide groups and the primary amines of the PEI, which enables faster aggregation at a lower temperature.

### Example 7: Stabilization of ELP-PEI Particle Sizes.

Crosslinking of the ELP particles was made possible by the addition of a copolymer such as PEI and allowed for the retention of the particle sizes and shapes, spherical and discs, after they are formed while minimizing the negative environments needed to maintain them (e.g. high NaCl concentrations). Examples of crosslinking is not limited and dependent upon surface properties that can be achieved with any chemistry reactive towards carboxylic acids, amines, and/or thiol groups such as: multi-functional aldehydes, trans-glutaminase, epoxide chemistry, carbodiimide chemistry, isocyanate chemistry, thiol-ene/yne chemistry, acrylates, methacrylates, and so forth. Purification of crosslinked particles was performed through standard methods for small molecule removal such as dialysis and low speed centrifugation.

The ELP-PEI copolymer solution samples were heated above their respective LCST in an oven. The LCSTs ranged from 22 to 37 °C. Glutaraldehyde was then added (10:1 molar ratio of aldehyde to ELP) and allowed to react with the ELP-PEI copolymer particles in the oven for 15 minutes. The samples underwent a thermo-cycling purification in a temperature-controlled centrifuge. For determining aggregation sizes of the cross-linked ELP-PEI copolymers and to ensure the crosslinking was successful the samples were cooled to 4 °C, and DLS was performed at a static temperature of 20 °C, which is below the LCST of the non-crosslinked particles.

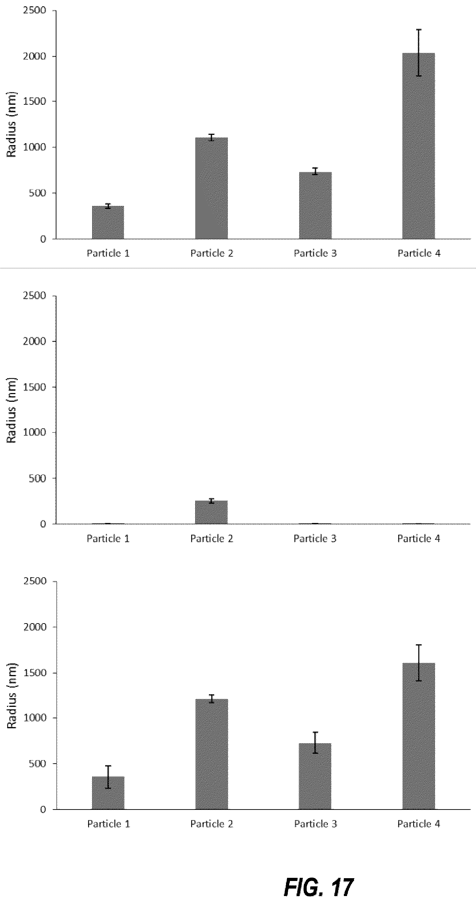

In Figure 17, DLS results showed the effect on non-crosslinked and crosslinked ELP-PEI particles below the Tt temperature. Figure 17 illustrates the size comparisons for non-crosslinked ELP-PEI particles above Tt (top), non-crosslinked below Tt (middle), and crosslinked particles below Tt (bottom). Crosslinked ELP-PEI particles regardless of size and how prepared retained their original diameter when lowered to a temperature below the ELP-PEI Tt, unlike non-crosslinked ELP-PEI particles below Tₜ the disaggregate. Additionally, particle size can be fixed through ELP-PEI linked to self-setting moieties, such as collagen as well as crosslinking of ELP-PEI linked moieties through charge interaction, such as alginate crosslinking using divalent calcium ions.

It will be apparent to those skilled in the art that various modifications and variations can be made in the present invention without departing from the scope or spirit of the invention. Other aspects of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A polypeptide-based particle, comprising:
copolymers of an elastin-like polypeptide (ELP) and polyethyleneimine (PEI),
wherein a plurality of the ELP and PEI copolymers are crosslinked such that the polypeptide-based particle is resistant to dissolution below a lower critical solution temperature (LCST) of the ELP.

2. The polypeptide-based particle of claim 1, wherein the ELP comprises about 5 to about 320 repeating units of the amino acid sequence VPGXG, where X is any amino acid except proline, such as where X is valine.

3. The polypeptide-based particle of claim 1, wherein the polypeptide-based particle further comprises a therapeutic agent.

4. The polypeptide-based particle of claim 3, wherein the therapeutic agent is selected from the group consisting of a drug, a gene, a protein, and a peptide, or wherein the therapeutic agent is coupled to the polypeptide-based particle with a drug binding domain.

5. The polypeptide-based particle of claim 1, wherein the PEI is coupled to an amino terminus or a carboxylic acid of the ELP.

6. The polypeptide-based particle of claim 1, wherein the ELP and PEI copolymers have a molecular weight ranging from 800 g/mol to 10,000 g/mol.

7. The polypeptide-based particle of claim 1, further comprising ELP that is not copolymerized with PEI, wherein a ratio of the ELP and PEI copolymers to ELP ranges from 1.2:98.8 to 15:85.
